# FASCICULE DE BREVET EUROPEEN

(11) **EP 3 310 282 B1**
(45) Date de publication et mention de la délivrance du brevet: **30.08.2023**
(21) Numéro de dépôt: 16739198.6
(22) Date de dépôt: 11.05.2016
(51) Int. Cl.: A61B 17/72, A61B 17/86

(54) **IMPLANT POUR LA FIXATION D'ÉLÉMENTS OSSEUX**
IMPLANTAT ZUR FIXATION VON KNOCHENELEMENTEN
IMPLANT FOR THE FIXATION OF BONE ELEMENTS

(30) Priorité: 11.05.2015 FR 1554199
(43) Date de publication de la demande: 25.04.2018
(73) Titulaire: Spine Arch Brevet, 67203 Oberschaeffolsheim (FR)
(72) Inventeur: FISCHER LOKOU, David François, 67203 Oberschaeffolsheim (FR)
(74) Mandataire: Mundel, Lysiane
(86) Numéro de dépôt international: PCT/FR2016/051116
(87) Numéro de publication internationale: WO 2016/181078

(56) Documents cités:
- EP-A1- 2 859 856
- WO-A1-2015/095353
- US-A1- 2009 319 043
- US-A1- 2015 012 048
- US-B1- 6 558 386

## Description

La présente invention a pour objet un nouvel implant à usage médical permettant la fixation d'au moins deux éléments osseux adjacents de manière rapide, fiable et peu invasive. Cet implant est un dispositif en deux parties distinctes formant vis une fois lesdites deux parties assemblées. Une méthode de fixation d'au moins deux éléments osseux adjacents mettant en oeuvre ce nouvel implant formant est également décrite.

Cette méthode est particulièrement adaptée pour la solidarisation d'au moins deux vertèbres en vue de la réalisation d'une arthrodèse ; ledit implant formant vis est alors de forme courbe ce qui le rend particulièrement bien adapté à la chirurgie du rachis.

Eu égards au vieillissement de la population et également au rythme de vie de la population d'actifs, les problèmes osseux génèrent ou font partie du tableau clinique de nombreuses pathologies. Par ailleurs les traumatismes liés à des accidents, chocs et chutes, peuvent également engendrer des problèmes au niveau osseux nécessitant une intervention chirurgicale. La possibilité de fixation d'éléments osseux entre eux afin de stabiliser ou de corriger une position donnée est donc un enjeu médical important. En effet, dans le domaine de la chirurgie du rachis en particulier le fait de pouvoir solidariser des éléments osseux afin de stabiliser des zones données et/ou de libérer des faisceaux nerveux comprimés est souvent la seule façon de soulager les patients.

Dans le domaine de la chirurgie orthopédique on utilise communément des plaques et des vis afin de stabiliser des zones osseuses adjacentes ou de leur conférer une position donnée. Les matériaux utilisés sont bien évidemment solides et de nombreuses contraintes mécaniques sont imposées par le matériau et le matériel. Pour permettre plus d'adaptation et autoriser plus de possibilités aux chirurgiens, différents dispositifs ont été mis en oeuvre afin de permettre une meilleure réponse chirurgicale pour chaque patient : vis chirurgicales en combinaison selon différents angles, plaques fixes, plaques articulées, cages, etc.

Dans le domaine particulier de la chirurgie du rachis, les chirurgiens utilisent en général des vis pédiculaires également appelées vis tulipes pour solidariser les vertèbres. Ces vis à usage médical sont des vis droites qui permettent l'arthrodèse, c'est-à-dire la fusion osseuse entre au moins deux vertèbres. Cette technique est communément utilisée en cas de fracture, de scoliose, d'instabilité et de douleurs importantes. Même si cette technique est très couramment pratiquée, la mise en place de vis pédiculaires demeure une procédure difficile qui est réservée aux chirurgiens spécialisés et est susceptible d'entraîner de faux trajets. Dans les cas de faux trajets, une ou plusieurs vis n'étant pas correctement placées, elle peuvent blesser les racines nerveuses ou présenter un défaut de solidité. Pour pallier cette éventualité de faux trajet, plusieurs systèmes sophistiqués d'aide au guidage sont utilisés par les chirurgiens, on citera notamment "la navigation" comprenant des repérages infra-rouge couplés à un scanner per opératoire. Ces systèmes de navigation permettent de diminuer le risque d'erreur de trajectoire. Cependant le facteur de risque d'erreur reste important car les arthrodèses pratiquées à ce jour nécessitent au minimum quatre vis pédiculaires et plus souvent six à huit vis pédiculaires.

Dans l'art antérieur, de nombreux documents décrivent la fixation de deux éléments osseux adjacents par des systèmes plus ou moins complexes et plus ou moins invasifs mettant en oeuvre des éléments métalliques et parfois des éléments pouvant adopter une forme courbe. Si l'on s'intéresse plus particulièrement à la fixation de vertèbres le brevet EP1176916 décrit par exemple un système de fixation du rachis comprenant un élément de liaison pouvant être arqué dont la longueur est établie de telle sorte qu'il s'étende entre deux vertèbres adjacentes sans protrusion sur les vertèbres. Cet élément de liaison qui n'est pas obligatoirement rigide, ni obligatoirement arqué, permet la fusion et/ou la stabilisation de vertèbres. L'implantation de plusieurs de ces éléments à l'intérieur d'un ou plusieurs alésages pratiqués dans des corps vertébraux est également envisagée. Selon EP1176916 les éléments de liaison sont constitués de métal, os, protéine morphogénique, composite en fibre de carbone, nitinol, matériau biodégradable ou encore une combinaison de ces matériaux. Un orifice d'insertion est pratiqué dans chaque corps vertébral adjacent et calculé afin que les orifices de sortie coïncident dans l'espace intervertébral des deux vertèbres adjacentes à fixer. La mise en place de cet élément de liaison dans sa version arquée nécessite la réalisation de plusieurs forages selon des abords distincts et très précis ce qui est complexe à réaliser in situ et facteur de risque pour le patient dans une zone d'opération difficile d'accès et très innervée.

On citera également le brevet EP1265541 qui décrit l'utilisation d'un implant spinal axial destiné à être inséré dans un alésage incurvé pratiqué au niveau caudal à travers le sacrum et s'étendant en direction céphalade à travers au moins deux corps vertébraux. L'alésage est pratiqué de façon à pouvoir insérer des implants allongés axiaux dans l'alignement d'une ligne visualisée transsacrale d'instrumentation/fusion (TASIF) depuis le niveau caudal vers la tête du patient. L'alésage et l'implant présentent une courbure pour répondre à la morphologie du rachis du patient dans la zone sacrolombaire. Cette méthode est invasive car si un étage supérieur est visé il faut quand même réaliser un alésage caudal afin d'atteindre la zone à implanter. L'implant présente en outre au moins un filetage pour venir en prise avec l'os du corps vertébral traversé pour maintenir l'implant en position et éviter une rétraction caudale de l'implant spinal axial. Cette méthode est couramment utilisée pour la chirurgie du rachis mais ne permet pas d'envisager la mise en place d'un implant qui ne soit pas axial. De plus elle est invasive et nécessite un matériel d'imagerie sophistiqué pour minimiser les risques au moment de la réalisation de l'alésage qui est obligatoirement en partie caudale à travers le sacrum, et de l'insertion de l'implant axial.

On citera également EP2859856 qui décrit l'utilisation d'une vis osseuse constituée de deux éléments distincts lui conférant une flexibilité longitudinale limitée. Elle est constituée d'une tige équipée d'une gorge dans laquelle un élément hélicoïdal est préalablement inséré. La gorge est conçue de telle sorte que cet élément hélicoïdal est apte à sensiblement glisser vers le haut ou vers le bas dans l'axe longitudinal de la vis une fois cette dernière en place ; ainsi en fonction des contraintes mécaniques imposées à la vis, elle pourra quelque peu s'adapter aux contraintes de son environnement. Cependant cette vis est droite. Elle est destinée à être insérée dans un os et éventuellement une vertèbre dans un abord pédiculaire. Du fait de sa flexibilité longitudinale elle est inapte à fixer deux éléments osseux entre eux. En outre les deux éléments de ce type de vis doivent être assemblés avant leur utilisation chez le patient.

WO2015/095353 décrit une vis transdiscale qui peut être de forme courbe constituée d'une tige et d'une gaine filetée.

Enfin le brevet FR2951370 par exemple décrit un implant qui permet une stabilisation instrumentée du rachis à l'aide d'une cage articulée qui vient combler l'espace intersomatique. Ceci est une méthode permettant de remplacer un disque intervertébral abimé et de fusionner deux vertèbres adjacentes. Ladite cage est maintenue dans la position souhaitée par des vis pédiculaires. Or il est bien connu de l'art antérieur que la mise en place de la cage et des vis, qui sont relativement nombreuses, nécessite un contrôle par imagerie de la zone d'implantation et surtout une grande expérience et une maîtrise certaine de la part du neurochirurgien qui devra éviter toute « fausse route » lors de l'installation de chaque vis pédiculaire afin de ne pas léser la zone à implanter. Cette technique et ce type d'implants sont couramment utilisés mais la technique de pose de ce type d'implants présente notamment les inconvénients liés au positionnement des vis pédiculaires décrits ci-dessus et imposent l'utilisation d'un matériel d'imagerie complexe et coûteux.

Ainsi les techniques utilisées à ce jour présentent plusieurs inconvénients dont la complexité des dispositifs à implanter, l'abord transpédiculaire pour les vis de fixation de ces dispositifs qui est relativement invasif et générateur d'effets secondaires (hémorragies, lésions nerveuses, etc.), la nécessité d'un suivi impliquant des techniques d'imagerie performantes, soit autant de facteurs de risques, de technicité et de coûts qui sont à prendre en considération.

La présence de nerfs, vaisseaux sanguins et autres tissus qui doivent nécessairement être préservés à l'occasion des chirurgies osseuses impose des voies d'accès et des abords limités au chirurgien. Les erreurs de trajectoires sont d'autant plus nombreuses que la ou les voies d'accès sont malaisées ou exiguës. Dans certains cas compte tenu de la configuration spatiale des éléments osseux à solidariser les techniques connues de l'art antérieur n'offrent aucune solution. Il existe donc un réel besoin de solutions techniques nouvelles ouvrant de nouvelles possibilités de fixation d'éléments osseux dans des configurations spécifiques.

La vis courbe est l'une de ces solutions, elle n'a cependant jamais été proposée à ce jour. En effet, la vis présente l'avantage de permettre la mise en place d'une fixation solide nécessitant une seule voie d'accès. La forme courbe permet quant à elle de travailler des voies d'accès offrant des angles de travail qui ne conviennent pas aux vis rectilignes connues de l'art antérieur.

La présente invention propose un implant chirurgical pour la fixation d'au moins deux éléments osseux adjacents comme revendiquée ci-dessous. Des caractéristiques additionnelles sont décrites dans les revendications dépendantes. Des méthodes de fixation d'au moins deux éléments osseux adjacents sont aussi décrites pour aider à la compréhension de l'invention mais ne font pas parties de l'invention revendiquée.

La présente invention propose un implant chirurgical simple qui permet de réaliser la fixation d'au moins deux éléments osseux adjacents, par exemple deux vertèbres adjacentes, sans risque de faux trajet, sans difficulté technique particulière et sans aide technique coûteuse autre qu'un simple appareil de radioscopie. Dans le cas de la chirurgie du rachis, contrairement à la technique mettant en oeuvre des vis pédiculaires, les implants selon la présente invention ne sont pas positionnés dans les pédicules vertébraux mais permettent de solidariser directement les corps vertébraux entre eux. Le dispositif est d'emblée plus solide que les vis pédiculaires couramment utilisées dans l'art antérieur. D'autre part les corps vertébraux étant volumineux, de l'ordre de 4 x 5 x 3 cm, le risque d'un trajet extracorporéal est très limité par rapport aux pédicules vertébraux qui font 1 × 0,8 cm et sont assez difficiles à identifier même avec des techniques d'imagerie performantes.

La présente invention permet donc une nouvelle méthode de fixation d'au moins deux éléments osseux adjacents, par exemple deux vertèbres adjacentes, sans risque de faux trajet et sans difficulté technique particulière pour un chirurgien. Cette méthode permet également un gain de temps considérable dans le cas de la stabilisation de plus de deux éléments osseux adjacents tels plusieurs étages vertébraux. De plus ce nouvel implant chirurgical qui une fois mis en place forme vis, est placé directement dans l'élément osseux, sans agrafes ni clous, ainsi une opération d'arthrodèse par exemple peut être réalisée rachis fermé entre les faisceaux nerveux et sans léser les nerfs.

Dans la présente invention, les termes suivants sont utilisés pour décrire le nouvel implant chirurgical et les éléments le constituant. Ils sont utilisés au pluriel comme au singulier. Par « *patient*» on entend un être humain ou un animal dont les symptômes ou la pathologie nécessitent l'intervention d'un chirurgien pour la solidarisation d'au moins deux éléments osseux entre eux. Par exemple un patient est un individu ayant subi une fracture de vertèbre ou présentant une scoliose, un tassement vertébral etc. la méthode proposée pour soulager les douleurs dudit patient étant la réalisation d'une arthrodèse, c'est-à-dire la fusion osseuse entre deux vertèbres.

Par *« élément osseux »* on entend des éléments osseux anatomiques nécessitant une intervention chirurgicale de stabilisation ou de réparation mais également tout implant qui remplace pour tout ou partie un élément osseux anatomique par exemple un implant en métal ou alliage de métal, céramique, polymère, ou tout autre matériau biocompatible.

Par « *courbe* » on entend une forme décrivant un arc plan ou en trois dimensions et non nécessairement régulier.

Par « tige » on entend un élément solide de forme cylindrique, plein ou creux, qui constitue le premier élément de l'implant selon l'invention, qui comprend sur tout ou partie de sa longueur un élément de guidage en forme de gorge hélicoïdale destiné à accueillir l'élément hélicoïdal. La tige peut être de forme droite, courbe ou mixte ; lorsqu'elle est mixte la tige présente une partie droite et une partie courbe sans que la tige ne présente obligatoirement de symétrie.

Par « *élément hélicoïdal »* on entend un élément solide plein de forme hélicoïdale, pouvant être flexible, destiné à être inséré le long d'une tige afin de former vis avec ladite tige en constituant notamment le filetage de la vis ainsi formée. Cet élément hélicoïdal comprendra au moins une âme et selon le mode de réalisation un filet, ce filet constituant le filet de l'implant formant vis.

Par « *vis »* on entend une tige cylindrique ou conique, faite d'une matière solide pouvant être flexible, et présentant un relief hélicoïdal ou une partie filetée servant à exercer une pression, notamment pour réaliser un assemblage.

Par « *filetage séparé »* on entend un filetage qui est destiné à former vis avec une tige une fois dans la position souhaitée mais qui n'est pas solidaire du ladite tige avant d'être dans la position souhaitée.

Par « *gorge hélicoïdale* » on entend une rainure hélicoïdale pratiquée sur tout ou partie de la face externe de la tige, et dont la forme permet le passage et le maintien en son sein de l'âme de l'élément hélicoïdal, âme dont la forme est choisie en fonction de celle de la gorge. Par exemple en section transversale la gorge est en forme de C, dont l'ouverture est orientée vers la surface externe de la tige, donc de profil fermé, ce qui empêche la sortie de l'âme de l'élément hélicoïdal. Selon un autre mode de réalisation en section transversale la gorge est de forme évasée, par exemple en « V ».

Par « *mouvement hélicoïdal »* on entend un mouvement formé d'une composante de rotation et d'une composante de translation. Dans le cas où le pas de l'hélice de l'élément hélicoïdal souhaité est suffisamment large, la mise en mouvement hélicoïdal peut être obtenue par une simple poussée en translation. À l'inverse dans le cas où le pas de l'hélice de l'élément hélicoïdal souhaité est suffisamment étroit la mise en mouvement hélicoïdal peut être obtenue en imprimant une simple rotation à l'élément considéré.

Par « *portion »* on entend une partie de forme sensiblement cylindrique de la tige.

L'invention porte un implant chirurgical pour la fixation d'au moins deux éléments osseux adjacents d'un patient, caractérisé en ce qu'il se compose d'une tige destinée à être insérée en premier dans un forage pratiqué dans les aux moins deux éléments osseux et d'au moins un élément hélicoïdal distinct destiné à être inséré en second sur ladite tige, l'ensemble mis en place formant une vis.

L'implant chirurgical selon l'invention est particulièrement adapté lorsque lesdits éléments osseux sont des vertèbres. L' implant chirurgical comprend en outre pratiqué sur la surface externe de la tige, autant d'éléments de guidage en forme de gorge hélicoïdale que d'éléments hélicoïdaux, lesdits éléments de guidage en forme de gorge hélicoïdale présentant chacun une ouverture sur tout ou partie de leur longueur en direction de l'élément osseux, et chacun desdits éléments hélicoïdaux comprend une âme de forme et de dimensions complémentaires à la forme et aux dimensions d'au moins un élément de guidage en forme de gorge hélicoïdale afin de pouvoir s'insérer et coulisser dans ladite gorge et un élément d'ancrage présent sur tout ou partie de la longueur l'âme et saillant par l'ouverture en direction de l'élément osseux.

Ladite gorge hélicoïdale peut être plus ou moins étroite. Ainsi dans un mode de réalisation de l'invention cette gorge peut être très ouverte et se présenter sous la forme d'une sorte de léger vallonnement à la surface de la tige, vallonnement dans lequel la partie formant l'âme de l'élément hélicoïdal, va se laisser guider et venir s'insérer, la partie non insérée venant au contact des éléments osseux et coincer l'élément hélicoïdal entre le fond de la gorge et l'élément osseux assurant l'ancrage de la vis. Dans ce mode de réalisation l'élément hélicoïdal est idéalement en coupe de forme cylindrique, ovoïde, ou toute autre forme géométrique autorisant son coulissement dans la gorge.

Dans un mode de réalisation préféré de l'invention présentant une gorge, au moins un des éléments d'ancrage est un filet. Ce filet peut être réalisé selon différents profils, notamment arrondi ou tranchant selon que l'on recherche un ancrage par appui sur l'élément osseux ou par insertion dans celui-ci.

Selon un mode spécifique de réalisation présentant une gorge, le au moins un filet n'est pas de hauteur constante tout de son long. Ainsi la hauteur du filet émergeant de l'ouverture pourra être variable. Une hauteur importante permettant d'assurer un ancrage dans un élément osseux plus friable et une hauteur moins importante permettant un ancrage dans un tissu osseux plus dur. L'invention pourra également être réalisée avec une absence de filet sur certaine partie de l'élément hélicoïdal notamment celles qui seront environnés d'une zone de tissus non-osseux une fois l'implant mis en place. Selon une autre caractéristique additionnelle le filet présente sur ses parois des éléments tels des lamelles ou des picots, éventuellement sécables, visant à ancrer ledit filet dans l'os sans possibilité de retour à moins d'y être contraint par un mouvement exercé par le chirurgien avec le couple nécessaire pour les plier ou les briser.

Dans les modes de réalisation présentant une gorge, la gorge n'est pas nécessairement pratiquée sur toute la longueur de la tige. Il peut notamment être envisagé de ne pas pratiquer de gorge sur la partie de la tige qui occupera la partie la plus profonde du forage, c'est à dire se situant à l'opposé de l'orifice pratiqué par le chirurgien pour réaliser le forage et insérer l'implant. Selon un mode de réalisation de l'invention la profondeur de la au moins une gorge hélicoïdale n'est pas constante tout de son long. Il peut ainsi être envisagé de pratiquer des gorges de profondeur variable en diminuant la profondeur de la gorge pour les parties de la vis qui, une fois celle-ci mise en place, seront situées dans les éléments osseux et en augmentant la profondeur de la gorge pour les parties de la vis traversant des tissus non-osseux. Cette dernière configuration permettant de faire saillir le filet de la gorge de manière plus ou moins importante selon les portions de la tige.

Dans une variation de l'invention plusieurs gorges parallèles sont pratiquées sur la tige pour accueillir autant d'éléments hélicoïdaux de manière à former, une fois les éléments mis en place, une vis à filetage multiple. Dans les modes de réalisation de l'invention présentant au moins une gorge, l'âme de l'élément hélicoïdal présente en coupe transversale perpendiculaire à l'axe longitudinal de cette âme une forme, par exemple en V, en U ou ronde, complémentaire de l'élément de guidage en forme de gorge hélicoïdale par exemple de forme évasée en V, droite en U ou sensiblement fermée en C. L'âme de l'élément hélicoïdal étant ajustée afin de permettre son coulissement au sein de ladite gorge, tout en contraignant ledit coulissement, c'est-à-dire en empêchant que l'âme de l'élément hélicoïdal n'adopte une autre conformation que celle mécaniquement imposée par la gorge et par l'outil de pose lui imprimant un mouvement hélicoïdal. Il doit être noté que la pression de l'élément osseux sur l'élément d'ancrage de l'élément hélicoïdal maintient ce dernier dans l'élément de guidage en forme de gorge. Dans un mode de réalisation préféré la gorge hélicoïdale présente un profil de forme évasée avec l'ouverture dirigée en direction de l'élément osseux à assembler. L'âme de l'élément hélicoïdal de forme complémentaire viendra s'insérer dans la gorge lors de la pose par le chirurgien et sera maintenue en place par les contraintes mécaniques imposées par la forme de la gorge, le filet venant s'insérer dans l'os et la pression exercée par l'outil de pose.

Pour une meilleure tenue de l'implant selon l'invention il pourra toutefois être recherché d'améliorer ce maintien, notamment si l'élément osseux est fragilisé par endroits. Aussi selon une caractéristique alternative la gorge hélicoïdale présente un profil en forme de C avec l'ouverture dirigée en direction de l'élément osseux à assembler. Bien entendu tout autre profil fermé similaire assurera la même fonction de maintien de l'âme de l'élément de guidage et présente une variante de l'invention. Le profil de la au moins une gorge hélicoïdale sera considéré comme fermé si en coupe transversale perpendiculaire à l'axe longitudinal de ladite au moins une gorge, la dimension de l'ouverture de la au moins une gorge est inférieure à la plus grande dimension de l'âme de l'au moins un élément hélicoïdal mesurée selon toute droite parallèle à ladite ouverture.

La forme de la tige détermine la forme de l'implant, ainsi dans un mode de réalisation particulier la tige est sensiblement droite et l'implant chirurgical formant vis est sensiblement droit ; dans un mode de réalisation préféré la tige est courbe et l'implant chirurgical formant vis une fois celle-ci mise en place est lui aussi courbe. Dans un mode de réalisation spécifique la tige est courbée dans différents plans. Selon une caractéristique additionnelle la forme de l'implant chirurgical formant vis selon l'invention est courbe. Selon une autre caractéristique additionnelle la forme de l'implant chirurgical est mixte, c'est-à-dire qu'elle présente une ou plusieurs portion droite et une ou plusieurs portion courbe.

Les matériaux utilisés pour la réalisation de l'implant chirurgical selon l'invention sont des matériaux solides biocompatibles par exemple de type métal ou alliage métallique, particulièrement le titane ou les alliages à base de titane et/ou l'acier ou les alliages à base d'acier, les céramiques à usage médical, les matériaux polymères à usage médical, lesdits matériaux étant envisagés seuls ou en combinaison. Selon une caractéristique additionnelle la tige et l'élément hélicoïdal sont réalisés en métal ou en un alliage à base de métal. Dans un mode de réalisation particulier, les propriétés mécaniques de la tige et de l'élément hélicoïdal distinct étant différentes, ces deux éléments sont réalisés dans des matériaux biocompatibles différents ; typiquement des métaux ou des alliages à base de métaux ayant des propriétés de flexibilité, d'élasticité, de dureté, d'étirement, de dilation différentes. Dans un autre mode de réalisation distinct la tige elle même est réalisée en dans des matériaux biocompatibles différents de manière à présenter selon ses tronçons des propriétés, notamment de flexibilité différentes. Bien entendu dans des modes de réalisation spécifiques il peut être envisagé différents traitement de surface de la tige, de l'âme de l'élément hélicoïdal ou du filet de ce dernier par exemple pour assurer la biocompatibilité, le tranchant, l'effet ressort, le coulissement ou au contraire l'adhérence de tel ou tel de ces éléments. Dans un mode de réalisation spécifique l'élément hélicoïdal est réalisé en matériaux présentant une élasticité tel que par exemple un acier trempé.

Lorsque la tige est réalisée dans un matériau rigide, le chirurgien doit pratiquer un forage au sein des éléments osseux à assembler dont la courbure est identique à celle de la tige. Lorsque la tige est réalisée dans un matériau solide mais flexible, la tige est susceptible de s'adapter à toute courbure du forage réalisé par le chirurgien selon des paramètres prédéfinis. Ainsi selon une caractéristique additionnelle la tige est réalisée dans un ou plusieurs matériaux biocompatible(s) flexible(s). La tige peut notamment avoir une forme de clou.

La simplicité de cet implant chirurgical le rend particulièrement adaptable à la morphologie des éléments osseux adjacents à fixer entre eux. En effet il est aisé de fabriquer « sur mesure » des implants chirurgicaux formant vis de forme sensiblement droite ou courbe, présentant une symétrie ou non par rapport à ladite courbe, etc. Toutes les formes sont autorisées dans la présente invention en fonction de la zone à implanter. Pour une implantation au niveau vertébral, on privilégiera par exemple un implant chirurgical de forme courbe afin de s'adapter à la morphologie des vertèbres et à leur articulation. La courbure est alors déterminée en fonction de la morphologie des éléments osseux à solidariser. La courbure de l'implant selon l'invention ne sera pas nécessairement plane et pourra présenter des torsions. La courbure de l'implant selon l'invention n'est pas nécessairement régulière. Lorsqu'elle est régulière et en l'absence de torsion, une partie courbe de l'implant selon l'invention décrira un arc typiquement compris entre 1 et 180 degrés, préférentiellement entre 15 et 90 degrés, plus préférentiellement entre 30 et 60 degrés.

D'une manière générale quelle que soit la forme de l'implant selon l'invention il pourra être envisagé de ne réaliser que certaines portions de la tige en un matériel flexible. Par exemple les parties flexibles pourront être prévues pour être positionnées à l'extérieur des éléments osseux à assembler, par exemple deux vertèbres de manière à former une sorte de charnière et à conserver une certaine souplesse à l'implant.

Selon un mode de réalisation spécifique, l'implant selon l'invention présente un dispositif distinct de blocage de l'élément hélicoïdal dans une position désirée sur la tige. Ce blocage peut s'effectuer par tout moyen mécanique connu de l'homme du métier, notamment par l'insertion d'un clou, d'une goupille ou d'une agrafe traversant les éléments à immobiliser, d'une douille ou d'un écrou les enserrant ou d'un filet intercalaire ou d'un coin faisant frein entre le fond de la gorge et l'élément de guidage. L'homme du métier pourra aussi obtenir ce blocage en utilisant des colles, résines, aimant ou ciments et tout autre mode de fixation biocompatibles.

Selon un mode de réalisation spécifique, l'implant selon l'invention présente au moins un dispositif d'accroche permettant la pose et le retrait de la tige et de l'élément hélicoïdal par le chirurgien à l'aide d'ancillaires adaptés. Ce dispositif d'accroche est selon un mode de réalisation privilégié situé sur une partie de la tige non insérée dans l'élément osseux. Selon une caractéristique privilégiée le dispositif d'accroche est situé sur une partie de l'extrémité de l'élément hélicoïdal non insérée dans l'élément osseux. Le dispositif d'accroche peut consister en tout moyen connu de l'homme du métier, tel que par exemple un filetage, un crochet, un anneau, une encoche, un méplat, un aimant, un relief, une empreinte, un retrait, une perforation.

L'invention permet la mise en oeuvre d'une méthode non revendiquée de fixation d'au moins deux éléments osseux, mettant en oeuvre l'implant chirurgical constitué d'une tige et d'au moins un élément hélicoïdal distinct, consistant à :
- pratiquer à l'aide d'un outil de forage un forage présentant les caractéristiques définies par le chirurgien en fonction des éléments osseux à solidariser, puis
- insérer la tige dans le forage ainsi pratiqué à l'aide d'un outil de pose, puis
- insérer chacun des au moins un élément hélicoïdaux distincts sur ladite tige à l'aide d'un outil de pose permettant d'imprimer isolément ou simultanément les différentes composantes du mouvement hélicoïdal audit au moins un élément hélicoïdal.

L' insertion de chacun des au moins un élément hélicoïdal distinct sur ladite tige se fait au niveau de l'élément de guidage en forme de gorge hélicoïdale et peut en outre comporter une étape terminale supplémentaire consistant à bloquer chacun des au moins un élément hélicoïdal sur la tige dans la position désirée à l'aide d'un dispositif de blocage. La dépose de l'implant chirurgical selon l'invention se fera en procédant aux mêmes opérations que la pose mais dans l'ordre inverse.

On comprendra aisément que la tige de l'implant selon l'invention peut être insérée comme un clou par simple percussion ou tout autre poussée sur son extrémité externe, sans qu'il ne soit nécessaire de lui imprimer un quelconque mouvement de rotation. La tige de l'implant selon l'invention peut même être insérée de la sorte dans un forage ou un logement présentant une certaine courbure, ce qui est rigoureusement impossible pour une vis traditionnelle rigide. Si le forage présente un rayon de courbure constant et se situant dans un seul plan, c'est-à-dire sans torsion, la tige de l'implant selon l'invention pourra être rigide mais devra avoir le même rayon de courbure que ledit forage. Si le rayon de courbure du forage n'est pas constant ou s'il présente une ou plusieurs torsions la tige de l'implant chirurgical selon l'invention devra nécessairement présenter une certaine souplesse.

On comprendra également aisément que la souplesse et/ou l'élasticité de l'élément hélicoïdal distinct selon l'invention permet son insertion par simple vissage le long de la tige, même courbe, en lui imprimant un mouvement hélicoïdal. L' insertion de chacun des au moins un élément hélicoïdal distinct sur ladite tige au niveau de l'élément de guidage en forme de gorge hélicoïdale permet une mise en place très précise.

Les caractéristiques de l'invention mentionnées ci-dessus, ainsi que d'autres, apparaîtront plus clairement à la lecture de la description suivante d'un exemple de réalisation, ladite description faisant référence aux figures jointes, parmi lesquelles :
la Fig. 1 représente une vue de la tige (2) constituant la partie de l'implant chirurgical (1) qui est insérée en premier dans la zone d'opération ;
la Fig. 2 représente une vue de la tige (2) constituant l'une des deux parties de l'implant chirurgical (1) au moment où elle va être insérée dans une vertèbre à l'aide d'un outil de forage et de pose ;
la Fig. 3 représente une vue de l'élément hélicoïdal (3) dans un mode de réalisation classique et dans un mode de réalisation particulier qui fait apparaître un filet (32) sur la partie de l'élément hélicoïdal (3) au contact de l'élément osseux et une âme (31) sur la partie interne de l'élément hélicoïdal (3) ;
la Fig. 4 représente la première partie de l'implant chirurgical (1) selon l'invention, à savoir la tige (2) une fois insérée entre deux vertèbres, on y voit un élément de guidage en forme de gorge hélicoïdale (4) pratiqué sur la surface externe de la tige (2) ;
la Fig. 5 représente l'insertion de la deuxième partie de l'implant chirurgical (1) au niveau de deux vertèbres adjacentes, on y distingue la tige (2) et l'élément hélicoïdal (3) qui coulisse le long de ladite tige (2) au sein du corps vertébral grâce à un outil de pose adéquat ;
la Fig. 6 représente l'implant chirurgical (1) une fois positionné entre deux vertèbres adjacentes, on y voit la tige (2) et l'élément hélicoïdal (3) dans leur position finale, formant ainsi une vis au sein de deux corps vertébraux adjacents.
   Ces figures de 1 à 6 montrent un exemple de mise en place d'un implant selon l'invention et décrivent la solidarisation de deux vertèbres adjacentes grâce à un implant chirurgical (1) tel que décrit ci-dessus. Le mode de réalisation illustré fait apparaître une tige (2) de forme courbe et un élément hélicoïdal (3) qui est inséré dans un élément de guidage en forme de gorge hélicoïdale (4).

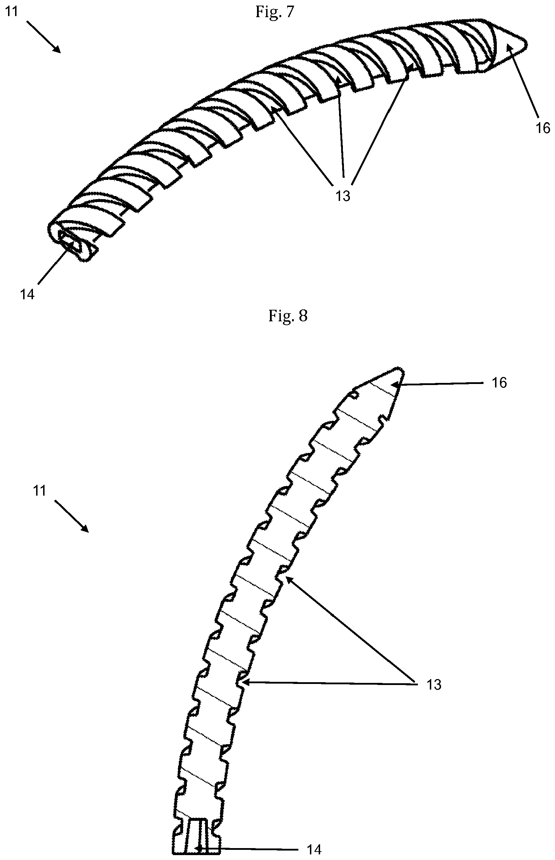

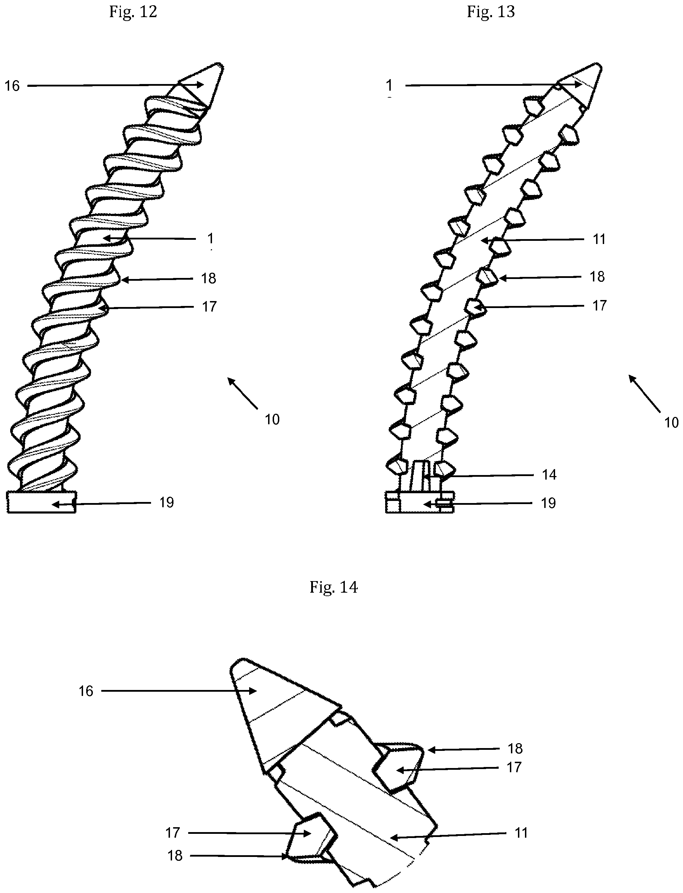

la Fig. 7 représente une vue isométrique de la tige (11) avec une double gorge (13) de l'implant courbe (10) de la Fig. 12;
la Fig. 8 représente une vue en coupe longitudinale de la tige (11) avec une double gorge (13) de l'implant courbe (10) de la Fig. 12;

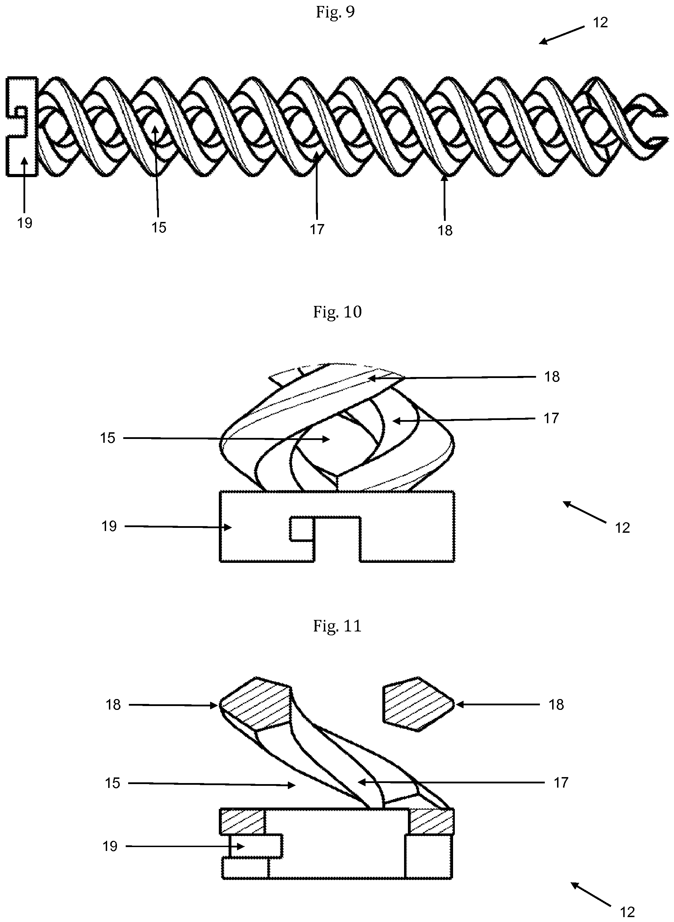

la Fig. 9 représente une vue isométrique de l'élément hélicoïdal (12) composé de deux hélices identiques, de l'implant courbe (10) de la Fig. 12 ;
la Fig. 10 représente une vue de face de l'extrémité proximale de l'élément hélicoïdal (12) avec son système d'ancrage (19) et la base de la double hélice de l'implant courbe (10) de la Fig. 12;
la Fig. 11 représente une vue en coupe longitudinale de l'extrémité proximale de la Fig. 10 ;
la Fig. 12 représente une vue de face d'un implant courbe (10) complet formant vis selon l'invention décrite en détails dans l'exemple ci-après;
la Fig. 13 représente une vue en coupe longitudinale de l'implant courbe (10) de la Fig. 12 ;
la Fig. 14 représente l'extrémité distale de l'implant courbe (10) de la figure 12 avec une tête (16) de forme conique.

Cette tige selon les Fig. 7 à 14 présente à une extrémité une tête de forme conique apte à pénétrer l'os et à l'autre extrémité une partie destinée à être fixée sur l'outil de pose. La taille de cette tête est typiquement de 6 mm entre sa base et son extrémité. La longueur développée de la tige est de 70 mm et son diamètre de 4 mm mesuré de fond de gorge à fond de gorge et de 6 mm en affleurement de gorge. L'arc de courbure de la tige s'inscrit dans un cercle de rayon de 90 mm, et décrit un angle de 44,6°.

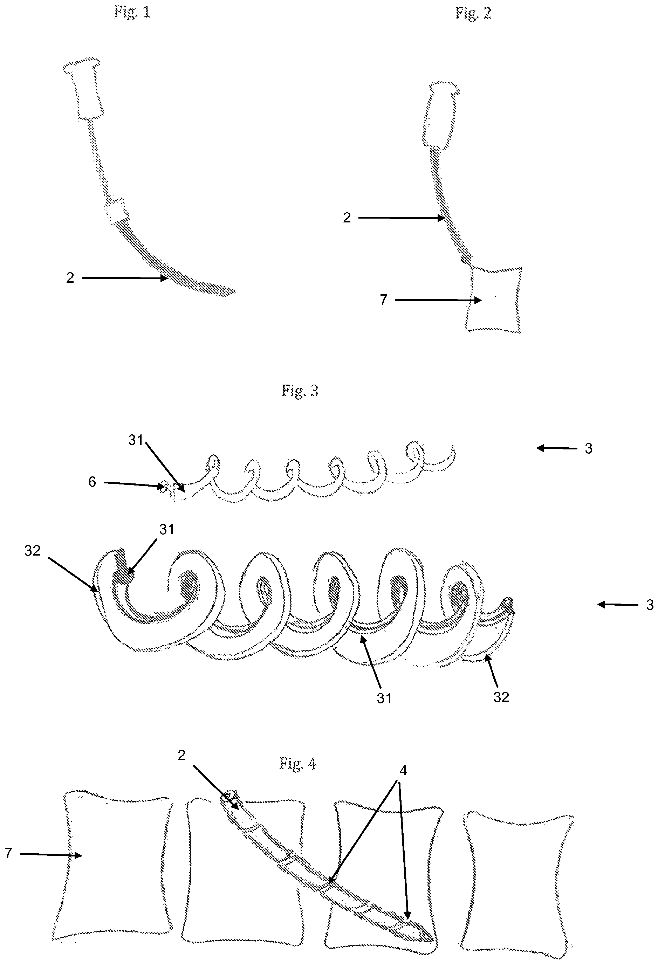

Les Fig. 1 et 2 illustrent le premier élément constituant l'implant chirurgical (1) selon l'invention, à savoir la tige (2) et un outil de pose adéquat. Ce premier élément est inséré à l'aide d'un outil de forage et de pose au sein de deux éléments osseux adjacents, ici deux vertèbres. Cette première étape est représentée en Fig. 2.

La Fig. 3 montre le détail du second élément constituant l'implant chirurgical (1) selon l'invention, l'au moins un élément hélicoïdal (3) distinct. Le premier schéma montre l'âme (31) de cet élément hélicoïdal (3) qui va venir coulisser dans l'élément de guidage en forme de gorge hélicoïdale (4) de la tige (2), ainsi qu'un dispositif d'accroche (6). Le second schéma montre l'élément hélicoïdal (3) constitué d'une âme (31) et d'un filet (32) venu de matière avec l'âme (31). Dans un mode de réalisation préféré l'élément hélicoïdal présente une extrémité distale qui va venir en affleurement ou en butée contre la base de la tête de forme conique sur l'extrémité distale de la tige et une extrémité proximale munie d'un dispositif de fixation par l'outil de pose. Typiquement le diamètre extérieur de l'élément hélicoïdal est de 10 mm et le diamètre intérieur est de 4 mm.

La Fig. 4 montre la tige (2) une fois positionnée au sein de deux corps vertébraux à solidariser. Cette tige (2) présente un élément de guidage en forme de gorge hélicoïdale (4) destinée à accueillir l'âme (31) de l'élément hélicoïdal (3) qui est dimensionnée de façon complémentaire à l'élément de guidage en forme de gorge hélicoïdale (4). L'étape suivante est la pose de l'élément hélicoïdal (3) à l'aide d'un outil de pose lui imprimant un mouvement hélicoïdal permettant son insertion autour de la tige (2).

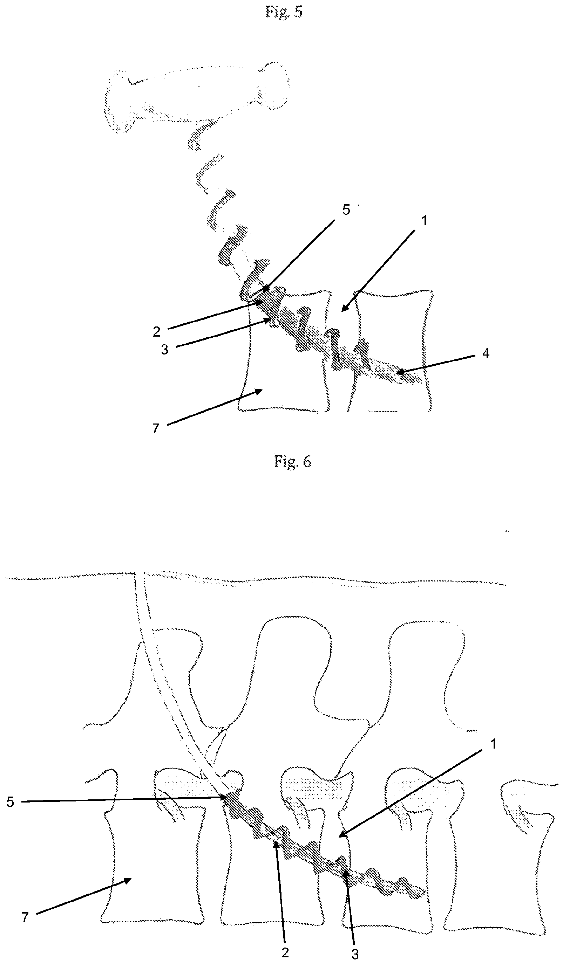

La Fig. 5 montre l'étape évoquée ci-dessus de la mise en place de l'élément hélicoïdal (3) sur la tige (2) au sein de l'élément de guidage en forme de gorge hélicoïdale (4) ; ladite tige (2) servant de guide.

La Fig. 6 montre l'implant chirurgical (1) dans sa position finale au sein de deux corps vertébraux dans notre exemple. L'élément hélicoïdal (3) est inséré le long de la tige (2) et bloqué dans sa position finale par un dispositif de blocage (5). Les pédicules des vertèbres ne sont pas affectés par la mise en place de l'implant chirurgical (1) ainsi on évite tout problème de faux trajet et/ou de lésion nerveuse ou vasculaire.

Dans un mode de réalisation particulier, la présente invention concerne un implant chirurgical courbe (1) pour la fixation d'au moins deux éléments osseux adjacents d'un patient, qui se compose d'une tige (2) destinée à être insérée en premier dans un forage pratiqué dans les au moins deux éléments osseux adjacents, et d'au moins un élément hélicoïdal (3) distinct destiné a être inséré en second sur ladite tige (2), l'ensemble mis en place formant vis.

Dans un second mode de réalisation particulier, la présente invention concerne un implant chirurgical courbe (10) pour la fixation d'au moins deux éléments osseux adjacents d'un patient, qui se compose d'une tige (2) destinée à être insérée en premier dans un forage pratiqué dans les au moins deux éléments osseux adjacents, et de deux éléments hélicoïdaux (3) distincts destinés a être insérés en second sur ladite tige (2), l'ensemble mis en place formant vis.

Ledit implant chirurgical courbe (1) se compose d'une tige (2) destinée à être insérée en premier dans un forage pratiqué dans les au moins deux éléments osseux et d'au moins un élément hélicoïdal (3) distinct destiné à être inséré en second, et :
- ladite tige (2) comprend pratiqués sur sa surface externe, autant d'éléments de guidage en forme de gorge hélicoïdale (4) que d'éléments hélicoïdaux (3), lesdits éléments de guidage en forme hélicoïdale (4) présentant chacun une ouverture en direction de l'élément osseux, et
- chacun des au moins un élément hélicoïdal (3) distinct comprend une âme (31) de forme et de dimensions complémentaires à la forme et aux dimensions d'au moins un élément de guidage en forme de gorge hélicoïdale (4) afin de pouvoir s'insérer et coulisser dans ledit au moins un élément de guidage en forme de gorge hélicoïdale (4) et un élément présent sur tout ou partie de la longueur de l'âme (31) et saillant par l'ouverture en direction de l'élément osseux,

l'ensemble mis en place formant une vis.

La mise en place d'un tel implant chirurgical (1) selon l'invention est typiquement réalisée à l'aide de deux outils distincts destinés à positionner très précisément les deux éléments constituant ledit implant chirurgical (1), à savoir la tige (2) et l'au moins un élément hélicoïdal (3) distinct au sein de l'élément de guidage en forme de gorge hélicoïdale (4). Un outil de forage permet de pratiquer un forage dans les deux éléments osseux à solidariser suivant un trajet déterminé par le chirurgien. Ensuite un premier outil permet la pose de la tige (2), puis un second outil de pose permet d'insérer l'au moins un élément hélicoïdal (3) autour de la tige au sein de l'élément de guidage en forme de gorge hélicoïdale (4) en lui imprimant un mouvement hélicoïdal. La tige (2) installée préalablement sert de guide à l'au moins un élément hélicoïdal (3). Selon une caractéristique additionnelle il peut être prévu un élément de butée afin de déterminer très précisément lorsque l'au moins un élément hélicoïdal (3) est dans sa position finale. Ledit élément de butée peut être situé soit à l'extrémité distale de la tige (2), soit à son extrémité proximale c'est-à-dire au niveau du point d'insertion de la tige (2) dans le premier élément osseux. Cet élément de butée peut également avoir une fonction de dispositif de blocage (5) lorsqu'il est situé en position proximale de l'implant chirurgical (1) une fois mis en place.

Lorsque le chirurgien le juge applicable en fonction des éléments osseux à assembler et du matériau constituant la tige (2), il peut être envisagé d'utiliser un outil de forage et de pose qui emploie la tige (2) de l'implant chirurgical (1) présentant une extrémité distale pointue comme tête de forage. Dans ce mode de réalisation il n'y a pas d'étape de forage préalable lorsque la méthode décrite est appliquée.

Selon une caractéristique additionnelle l'implant chirurgical (1) présente un dispositif d'accroche (6) afin que le chirurgien puisse en assurer aisément la pose et le retrait. Ce point d'accroche (6) est situé idéalement au niveau de l'extrémité proximale de l'implant chirurgical (1) c'est-à-dire au niveau du point d'insertion de la tige (2) dans le premier élément osseux. Le retrait de l'implant chirurgical (1) ou dépose se fait en procédant aux mêmes opérations que la pose mais dans l'ordre inverse.

Dans un mode de réalisation préféré l'implant chirurgical courbe (1) présente une tige (2) et au moins un élément hélicoïdal (3) distinct destiné à être inséré sur ladite tige (2) formant ainsi une vis de longueur développée comprise entre 17,5 mm et 280 mm, préférentiellement 35 mm et 140 mm, plus préférentiellement entre 50 mm et 90 mm, et s'inscrivant dans un arc compris entre 1 et 180 degrés, préférentiellement entre 15 et 90 degrés, plus préférentiellement entre 30 et 60 degrés.

Afin de conférer plus de solidité à l'implant il peut être prévu des éléments en relief, tels des picots, sur les parois du filet (32) de l'au moins un élément hélicoïdal (3) orientés de manière adéquate afin d'opposer une résistance au dévissage de la tige (2) et de l'élément hélicoïdal (3) formant vis après sa mise en place par le chirurgien.

Dans un mode de réalisation préféré l'invention concerne un implant chirurgical (1) pour la fixation d'au moins deux vertèbres adjacentes d'un patient, qui se compose d'une tige (2) courbe destinée à être insérée en premier dans un forage présentant la même courbure pratiqué dans les au moins deux vertèbres et d'un élément hélicoïdal (3) distinct destiné à être inséré en second, et que :
- ladite tige (2) comprend pratiquée sur sa surface externe, autant d'éléments de guidage en forme de gorge hélicoïdale (4) que d'éléments hélicoïdaux (3), lesdits éléments de guidage en forme hélicoïdale (4) présentant chacun une ouverture en direction des vertèbres, et
- chacun desdits éléments hélicoïdaux (3) comprend une âme (31) de forme et de dimensions complémentaires à la forme et aux dimensions du au moins un élément de guidage en forme de gorge hélicoïdale (4) afin de pouvoir s'insérer et coulisser dans ledit au moins un élément de guidage en forme de gorge hélicoïdale (4) et un élément présent sur tout ou partie de la longueur de l'âme (31) et saillant par l'ouverture en direction des vertèbres,

l'ensemble mis en place formant une vis courbe.

Dans un mode de réalisation particulier l'implant chirurgical (1) qui une fois mis en place forme vis courbe est destiné à la chirurgie du rachis et décrit un arc compris entre 30 et 60 degrés, préférentiellement entre 40 et 50 degrés pour une longueur développée de l'implant (10) comprise entre 35 mm et 140 mm.

Dans un mode de réalisation de l'invention, l'au moins un élément d'ancrage en forme de filet (32) émerge de l'élément de guidage en forme de gorge hélicoïdale (4) dans lequel il est inséré sur une hauteur comprise en direction de l'élément osseux typiquement entre 1/2 et 1/20^{éme} du diamètre de la tige (2). Dans un autre mode particulier de réalisation de l'invention le filet (32) n'est pas continu ou n'est pas réalisé sur la totalité de la longueur de l'âme (31). Dans un autre mode de réalisation de l'invention la profondeur de l'élément de guidage en forme de gorge hélicoïdale (4) n'est pas constante et présente des portions où elle est supérieure à la dimension de l'âme (31) s'inscrivant dans cette profondeur de manière à permettre de moduler la hauteur du filet (32) émergeant de l'élément de guidage en forme de gorge hélicoïdale (4) pour que celle-ci soit importante dans les portions de l'implant (1) qui sont situées dans un élément osseux et moins importante voir nulle pour les portions de l'implant qui traversent des tissus non osseux.

Dans un mode particulier de réalisation de l'invention plusieurs éléments de guidage en forme de gorges hélicoïdales (4) parallèles les unes aux autres sont pratiqués sur la surface externe de la tige (2). Une telle réalisation permet d'adopter un pas d'hélice important facilitant le coulissement de l'âme (31) du au moins un élément hélicoïdal (3) dans lesdits éléments de guidage en forme de gorges hélicoïdales (4) tout en préservant la longueur total de l'élément d'ancrage (32).

Une méthode non revendiquée de fixation d'au moins deux vertèbres adjacentes qui met en oeuvre un implant chirurgical (1, 10) tel que décrit ci-dessus, composé d'une tige (2, 11) et d'au moins un élément hélicoïdal (3, 12) distinct, consiste à :
- pratiquer à l'aide d'un outil de forage un forage présentant les caractéristiques définies par le chirurgien en fonction des vertèbres à solidariser, puis
- insérer la tige (2, 11) dans le forage ainsi pratiqué à l'aide d'un outil de pose sous radioscopie médicale, puis
- insérer chacun des au moins un élément hélicoïdal (3, 12) distinct sur ladite tige (2, 11) à l'aide d'un outil de pose permettant d'imprimer isolément ou simultanément les différentes composantes de mouvement hélicoïdal audit au moins un élément hélicoïdal (3), et optionnellement
- bloquer l'élément hélicoïdal (3, 12) sur la tige (2, 11) dans la position désirée à l'aide d'un dispositif de blocage (5) .

Selon une caractéristique additionnelle le blocage en position finale est réalisé grâce à un dispositif de blocage (5) et/ou élément de butée qui est placé soit en position distale, soit en position proximale de l'implant chirurgical (1). Selon un mode de réalisation particulier l'implant chirurgical présente un dispositif de blocage (5) de l'élément hélicoïdal (3) distinct dans une position désirée sur la tige (2). Le dispositif de blocage (5) évite au deux éléments de l'implant chirurgical (1) de bouger l'un par rapport à l'autre. Selon une caractéristique additionnelle le dispositif de blocage (5) est situé en position proximale de l'implant (1) et consiste en une bague de blocage réalisée en un matériau biocompatible.

Cette méthode est particulièrement adaptée à la fixation de plusieurs vertèbres adjacentes chez un patient présentant un problème lombaire nécessitant l'intervention d'un chirurgien car elle permet d'éviter l'insertion de vis pédiculaires selon les techniques utilisées actuellement, ce qui est moins invasif et limite grandement les effets secondaires liés à l'opération de fixation des vertèbres. De plus, l'opération peut être envisagée rachis fermé sous contrôle radioscopique, en passant entre les faisceaux nerveux et sans léser les nerfs.

Dans le cas de l'implantation du rachis chez un patient adulte, l'implant chirurgical (1, 10) selon l'invention est de forme courbe décrivant un arc compris entre 30 et 60 degrés, préférentiellement entre 40 et 50 degrés pour une longueur développée de l'implant (1, 10) compris entre 35 mm et 140 mm. Dans un premier temps un forage est réalisé avec un premier outil de forage. Ensuite un outil de pose est employé afin de placer la tige (2, 11) depuis la corticale à travers la partie tendre du premier corps vertébral et enfin vers le second corps vertébral à fixer. Dans un second temps l'au moins un élément hélicoïdal (3, 12) distinct est inséré le long de la tige (2, 11) à l'aide d'un ancillaire adapté permettant son insertion en lui imprimant un mouvement hélicoïdal, la tige (2, 11) préalablement positionnée dans le forage lui servant de guide ; c'est plus précisément l'âme (31, 17) de l'au moins un élément hélicoïdal (3, 12) qui vient coulisser au sein de l'au moins un élément de guidage en forme de gorge hélicoïdale (4, 13). L'âme (31, 17) de l'au moins un élément hélicoïdal (3, 12) est insérée jusqu'à atteindre une position finale dans laquelle elle va être bloquée par un dispositif de blocage (5) situé en partie proximale de l'implant (1).

Typiquement pour un patient adulte, la tige (2) a un diamètre de 6,0 mm et le filet (32) de l'élément hélicoïdal (3) distinct occupe un espace de 2,0 mm de part et d'autre de la tige (2). L'ensemble de l'implant chirurgical (1) nécessite donc un forage de l'ordre de 10,0 mm de diamètre en tout au sein des deux vertèbres adjacentes à fixer chez un patient adulte.

Dans un mode de réalisation particulier l'implant chirurgical (1, 10) présente une longueur développée comprise entre 35 mm et 140 mm et s'inscrit dans un arc de cercle compris entre 15 degrés et 90 degrés.

La présente invention porte également sur des trousses contenant le matériel nécessaire à la mise en oeuvre de la méthode décrite ci-dessus, à savoir un implant chirurgical (1, 10) dans l'un des modes des réalisation détaillés ci-dessus, un outil de forage, un outil de pose de la tige (2, 11) et un outil de pose de l'élément hélicoïdal (3, 12) permettant son insertion en lui imprimant un mouvement hélicoïdal; ces trois outils formant un kit d'ancillaires.

Dans un mode de réalisation préféré la trousse comprend un implant chirurgical (1, 10) de forme courbe et un kit d'ancillaires.

### Exemple 1 : implant chirurgical pour la fixation de vertèbres

Le présent exemple décrit un implant chirurgical selon l'invention parfaitement adapté à la solidarisation de deux vertèbres chez un patient adulte.

La tige (11) représentée en Fig. 7 est de forme courbe dans un plan, de longueur développée de 70 mm décrivant un arc de 44,6° et de 90 mm de rayon. La tige (11) a un diamètre de 6 mm et présente deux gorges hélicoïdales (13) identiques et équidistantes sur sa surface externe, ladite gorge ayant une profondeur de 1 mm. L'extrémité proximale de la tige (11) est pourvue d'un système d'ancrage ou d'accroche (14) à l'outil de pose. Son extrémité distale est équipée d'une tête de forme conique (16) destinée à favoriser la pénétration de la tige dans le forage préalablement réalisé par le chirurgien dans les vertèbres à solidariser. La gorge hélicoïdale (13) présente un profil de forme évasée comme cela est illustré par la Fig. 8 qui représente une coupe longitudinale de la tige (11) faisant clairement apparaître le profil évasé de la gorge (13) dont les parois forment un angle de 30° l'une par rapport à l'autre. La distance entre les extrémités supérieures des parois est de 2 mm. Cette gorge (13) s'étend jusqu'à la base de la tête conique (16). La tête (16) a une longueur de 6 mm entre son extrémité et sa base qui a un diamètre de 6 mm correspondant à l'épaisseur de la tige (11). La tige (11) présente à son extrémité proximale un dispositif d'accroche (14) à l'outil de pose. C'est typiquement un dispositif de fixation à baïonnette.

La Fig. 9 présente en vue de face un élément hélicoïdal (12) constitué de deux hélices donc de deux éléments hélicoïdaux ayant le même pas, des âmes (17) et des filets (18) de dimensions et de formes identiques. Ce double élément hélicoïdal (12) est destiné à être inséré en rotation sur la tige (11) au sein de la double gorge hélicoïdale (13) de forme et de dimensions complémentaires. Le double élément hélicoïdal (12) a une longueur de 67 mm entre son extrémité proximale et son extrémité distale. Son diamètre extérieur est de 10 mm et la lumière intérieure (15) a un diamètre de 4 mm.

La Fig. 10 décrit en détails la forme de l'âme (17) complémentaire de la forme évasée de la gorge hélicoïdale (13) et le filet (18) est saillant selon un angle aigu afin de pénétrer l'os et de s'y fixer solidement une fois l'implant (10) en place. L'accroche de l'élément hélicoïdal (13) à l'outil de pose est assurée par un dispositif d'accroche (19) à baïonnette. L'âme (17) et filet (18) de l'élément hélicoïdal (12) constituent les dispositifs de guidage et d'ancrage de l'élément hélicoïdal (13) au sein de la gorge hélicoïdale (14) d'une part et de la paroi osseuse d'autre part.

La Fig. 11 montre un plan de coupe de la Fig. 10 ainsi on y voit la forme des âmes de l'élément hélicoïdal double (17) complémentaires de la forme évasée des gorges hélicoïdales (13). La dimension de l'élément hélicoïdal, de la base de l'âme au sommet du filet, est de 3mm.

L'implant courbe (10) est représenté en vue longitudinale en Fig. 12. On y voit de haut en bas la tête conique (16) de la tige (11), les deux éléments hélicoïdaux (12) chacun avec son filet (18), chaque âme (17) étant insérée respectivement dans une gorge hélicoïdale (13), et le dispositif d'accroche (19) permettant la pose et la dépose de l'implant (10). La Fig. 13 est une vue en coupe longitudinale de l'implant. Enfin la Fig. 14 représente une vue en coupe longitudinale de l'extrémité distale de l'implant courbe (10) et notamment la tête conique (16). On y voit également la forme pentagonale des éléments hélicoïdaux (12) complémentaire de la forme évasée des gorges hélicoïdales (13) et le filet (18) destiné à pénétrer la surface osseuse pour y ancrer solidement l'implant courbe (10) .

### Exemple 2 : implant chirurgical pour la fixation de vertèbres.

Le présent exemple décrit un implant chirurgical de grande taille. Celui-ci reprend les éléments techniques de l'implant (10) selon l'agencement détaillé ci-dessus dans l'exemple 1 et a une longueur développée de 140 mm décrivant un arc de 89,1° et de 90 mm de rayon.

### Exemple 3 : implant chirurgical pour la fixation de vertèbres.

Le présent exemple décrit un implant chirurgical de petite taille. Celui-ci reprend les éléments techniques de l'implant (10) selon l'agencement détaillé ci-dessus dans l'exemple 1 et a une longueur développée de 35 mm décrivant un arc de 22° et de 90 mm de rayon.

### Exemple 4 :

Le tableau ci-dessous décrit la correspondance entre longueur développée de l'implant chirurgical (1, 10) selon l'invention et la valeur de l'angle de l'arc en fonction du rayon du cercle dans lequel s'inscrit ledit arc.

L'homme de l'art n'aura aucun problème à choisir une taille pour l'implant et une courbure adaptée en fonction de la taille et de la géométrie des éléments osseux à solidariser.

**Table 1 : valeur de l'angle (en °) de l'arc en fonction de la longueur développée de l'implant et du rayon du cercle pour une courbure constante.**

| | Rayon (mm) | | | | |
|---|---|---|---|---|---|
| | R30 | R60 | R90 | R120 | R150 |
| Longueur (mm) | | | | | |
| L 20 | 38,2 | 19, 1 | 12,7 | 9, 5 | 7,6 |
| L 35 | 66,8 | 33,4 | 22,3 | 16,7 | 13,4 |
| L 70 | 133,7 | 66,8 | 44,5 | 33,4 | 26,7 |
| L 90 | 171,9 | 85,9 | 57,3 | 43,0 | 34,4 |
| L 110 | | 105,0 | 70,0 | 52,5 | 42,0 |
| L 120 | | 114,6 | 76,4 | 57,3 | 45,9 |
| L 140 | | 133,7 | 89,1 | 66,8 | 53,5 |
| L 160 | | 152,8 | 101,9 | 76,4 | 61,1 |
| L 180 | | 172, 0 | 114,6 | 86,0 | 68, 8 |

## Revendications

1. Implant chirurgical (1) pour la fixation d'au moins deux éléments osseux adjacents d'un patient, **caractérisé en ce qu'**il se compose d'une tige courbe (2) destinée à être insérée en premier dans un forage pratiqué dans les aux moins deux éléments osseux et d'au moins un élément hélicoïdal (3) distinct destiné à être inséré en second sur ladite tige, l'ensemble mis en place formant une vis ;_et que
- l'élément hélicoïdal est un solide plein de forme hélicoïdale qui comprend au moins une âme (31) et un filet (32) ;
- ladite tige (2) comprend pratiqués sur sa surface externe, autant d'éléments de guidage en forme de gorge hélicoïdale (4) que d'éléments hélicoïdaux (3), lesdits éléments de guidage en forme de gorge hélicoïdale (4) présentant chacun une ouverture sur tout ou partie de leur longueur en direction de l'élément osseux, et
- chacun desdits au moins un élément hélicoïdal (3) comprend une âme (31) de forme et de dimensions complémentaires à la forme et aux dimensions du au moins un élément de guidage en forme de gorge hélicoïdale (4) afin de pouvoir s'insérer et coulisser dans ladite gorge (4) et un élément d'ancrage présent sur tout ou partie de la longueur de l'âme (31) et saillant par l'ouverture en direction de l'élément osseux.

2. Implant chirurgical (1) selon la revendication 1 **caractérisé en ce que** au moins un des éléments d'ancrage est un filet (32).

3. Implant chirurgical (1) selon l'une quelconque des revendications 1 à 2 **caractérisé en ce que** la profondeur de la au moins une gorge hélicoïdale (4) n'est pas constante tout de son long.

4. Implant chirurgical (1) selon l'une quelconque des revendications 1 à 3, **caractérisé en ce que** la tige (2) est réalisée en un ou plusieurs matériaux biocompatibles qui sont flexibles.

5. Implant chirurgical (1) selon l'une quelconque des revendications 1 à 4, **caractérisé en ce que** uniquement certaines des portions de la tige (2) sont flexibles.

6. Implant chirurgical (1) selon l'une quelconque des revendications 1 à 5, **caractérisé en ce qu'**il présente un dispositif distinct de blocage (5) du au moins un élément hélicoïdal (3) dans une position désirée sur la tige (2).

7. Implant chirurgical (1) selon l'une quelconque des revendications 1 à 6, **caractérisé en ce qu'**il présente au moins un dispositif d'accroche (6) permettant la pose et le retrait de la tige (2) et du au moins un élément hélicoïdal (3) par le chirurgien à l'aide d'ancillaires adaptés.

8. Implant chirurgical (1) selon l'une quelconque des revendications précédentes, **caractérisé en ce que** sa longueur développée est comprise entre 35 mm et 140 mm et s'inscrit dans un arc de cercle compris entre 15 degrés et 90 degrés.

9. Implant chirurgical (10) pour la fixation d'au moins deux vertèbres adjacentes d'un patient, **caractérisé en ce qu'**il se compose
- d'une tige courbe (11) qui présente au moins une gorge hélicoïdale (13), ladite tige courbe (11) étant destinée à être insérée en premier dans un forage pratiqué dans les aux moins deux vertèbres, et
- d'au moins un élément hélicoïdal (12) distinct destiné à être inséré en second sur ladite tige au sein de la au moins une gorge hélicoïdale (13), et dont l'âme (17) présente une forme complémentaire à la forme de la gorge hélicoïdale (13),
l'ensemble mis en place formant une vis.

10. Implant chirurgical (10) selon la revendication 9, **caractérisé en ce que** la tige courbe (11) présente en outre une extrémité distale qui porte une tête (16) et une extrémité proximale équipée d'un dispositif d'accroche (14); et que l'au moins un élément hélicoïdal (12) présente en outre un filet (18) et son extrémité proximale est équipée d'un dispositif d'accroche (19) ; l'au moins un élément hélicoïdal (12) étant destiné à être inséré en rotation sur la tige une fois mise en place.

11. Implant chirurgical (10) pour la fixation d'au moins deux vertèbres adjacentes d'un patient selon l'une quelconque des revendications 9 ou 10, **caractérisé en ce qu'**il se compose
- d'une tige courbe (11) qui présente deux gorges hélicoïdales (13), ladite tige courbe (11) étant destinée à être insérée en premier dans un forage pratiqué dans les aux moins deux vertèbres, et
- de deux éléments hélicoïdaux (12) distincts destiné à être inséré en second sur ladite tige au sein des deux gorges hélicoïdales (13), et dont les âmes (17) présentent une forme complémentaire à la forme des gorges hélicoïdales (13),
l'ensemble mis en place formant une vis.

12. Implant chirurgical (10) selon l'une quelconque des revendications 9 à 11, **caractérisé en ce que** sa longueur développée est comprise entre 35 mm et 140 mm et s'inscrit dans un arc de cercle compris entre 15 degrés et 90 degrés.

## Patentansprüche

1. Chirurgisches Implantat (1) zur Fixation von mindestens zwei benachbarten Knochenelementen eines Patienten, **dadurch gekennzeichnet, dass** es sich aus einem gekrümmten Schaft (2), der dazu bestimmt ist, als erster in eine Bohrung, die in den mindestens zwei Knochenelementen ausgebildet ist, eingeführt zu werden, und aus mindestens einem separaten spiralförmigen Element (3) zusammensetzt, das dazu bestimmt ist, als zweites auf dem Schaft eingeführt zu werden, wobei die eingesetzte Einheit eine Schraube bildet; und dass
- das spiralförmige Element ein massiver Festkörper mit Spiralform ist, der mindestens einen Kern (31) und ein Gewinde (32) umfasst;
- der Schaft (2) an seiner Außenfläche ausgebildet ebenso viele Führungselemente in Form einer spiralförmigen Nut (4) wie spiralförmige Elemente (3) umfasst, wobei die Führungselemente in Form einer spiralförmigen Nut (4) jeweils eine Öffnung über ihre gesamte oder einen Teil ihrer Länge in Richtung des Knochenelements aufweisen, und
- jedes des mindestens einen spiralförmigen Elements (3) einen Kern (31) mit einer Form und Abmessungen, die zu der Form und den Abmessungen des mindestens einen Führungselements in Form einer spiralförmigen Nut (4) komplementär sind, um in die Nut (4) eingeführt und in dieser gleiten zu können, und ein Verankerungselement umfasst, das auf der gesamten oder einem Teil der Länge des Kerns (31) vorhanden ist und durch die Öffnung in Richtung des Knochenelements vorsteht.

2. Chirurgisches Implantat (1) nach Anspruch 1, **dadurch gekennzeichnet, dass** mindestens eines der Verankerungselemente ein Gewinde (32) ist.

3. Chirurgisches Implantat (1) nach einem der Ansprüche 1 bis 2, **dadurch gekennzeichnet, dass** die Tiefe der mindestens einen spiralförmigen Nut (4) nicht über ihre gesamte Länge konstant ist.

4. Chirurgisches Implantat (1) nach einem der Ansprüche 1 bis 3, **dadurch gekennzeichnet, dass** der Schaft (2) aus einem oder mehreren biokompatiblen Materialien hergestellt ist, die elastisch sind.

5. Chirurgisches Implantat (1) nach einem der Ansprüche 1 bis 4, **dadurch gekennzeichnet, dass** nur einige der Abschnitte des Schafts (2) elastisch sind.

6. Chirurgisches Implantat (1) nach einem der Ansprüche 1 bis 5, **dadurch gekennzeichnet, dass** es eine separate Vorrichtung zum Sperren (5) des mindestens einen spiralförmigen Elements (3) in einer gewünschten Position auf dem Schaft (2) aufweist.

7. Chirurgisches Implantat (1) nach einem der Ansprüche 1 bis 6, **dadurch gekennzeichnet, dass** es mindestens eine Einhängevorrichtung (6) aufweist, die das Einbringen und Entfernen des Schafts (2) und des mindestens einen spiralförmigen Elements (3) durch den Chirurgen mit Hilfe von geeigneten Hilfsmitteln ermöglicht.

8. Chirurgisches Implantat (1) nach einem der vorstehenden Ansprüche, **dadurch gekennzeichnet, dass** seine abgewickelte Länge zwischen 35 mm und 140 mm beträgt und in einem Kreisbogen zwischen 15 Grad und 90 Grad verläuft.

9. Chirurgisches Implantat (10) zur Fixation von mindestens zwei benachbarten Wirbeln eines Patienten, **dadurch gekennzeichnet, dass** es sich zusammensetzt
- aus einem gekrümmten Schaft (11), der mindestens eine spiralförmige Nut (13) aufweist, wobei der gekrümmte Schaft (11) dazu bestimmt ist, als erster in eine Bohrung, die in den mindestens zwei Wirbeln ausgebildet ist, eingeführt zu werden, und
- aus mindestens einem separaten spiralförmigen Element (12), das dazu bestimmt ist, als zweites innerhalb der mindestens einen spiralförmigen Nut (13) auf dem Schaft eingeführt zu werden, und dessen Kern (17) eine Form aufweist, die zu der Form der spiralförmigen Nut (13) komplementär ist, wobei die eingesetzte Einheit eine Schraube bildet.

10. Chirurgisches Implantat (10) nach Anspruch 9, **dadurch gekennzeichnet, dass** der gekrümmte Schaft (11) weiter ein distales Ende aufweist, das einen Kopf (16) trägt, und ein proximales Ende, das mit einer Einhängevorrichtung (14) versehen ist; und dass das mindestens eine spiralförmige Element (12) weiter ein Gewinde (18) aufweist und sein proximales Ende mit einer Einhängevorrichtung (19) versehen ist; wobei das mindestens eine spiralförmige Element (12) dazu bestimmt ist, drehend auf dem Schaft, sobald dieser eingesetzt wurde, eingeführt zu werden.

11. Chirurgisches Implantat (10) zur Fixation von mindestens zwei benachbarten Wirbeln eines Patienten nach einem der Ansprüche 9 oder 10, **dadurch gekennzeichnet, dass** es sich zusammensetzt
- aus einem gekrümmten Schaft (11), der zwei spiralförmige Nuten (13) aufweist, wobei der gekrümmte Schaft (11) dazu bestimmt ist, als erster in eine Bohrung, die in den mindestens zwei Wirbeln ausgebildet ist, eingeführt zu werden, und
- aus zwei separaten spiralförmigen Elementen (12), die dazu bestimmt sind, als zweites innerhalb der zwei spiralförmigen Nuten (13) auf dem Schaft eingeführt zu werden, und deren Kerne (17) eine Form aufweisen, die zu der Form der spiralförmigen Nuten (13) komplementär ist, wobei die eingesetzte Einheit eine Schraube bildet.

12. Chirurgisches Implantat (10) nach einem der Ansprüche 9 bis 11, **dadurch gekennzeichnet, dass** seine abgewickelte Länge zwischen 35 mm und 140 mm beträgt und in einem Kreisbogen zwischen 15 Grad und 90 Grad verläuft.

## Claims

1. Surgical implant (1) for fixing of at least two adjacent bone elements of a patient, **characterized in that** it consists of a curved rod (2) intended to be inserted first into a borehole made in the at least two bone elements and at least one separate helical element (3) intended to be inserted secondly on said rod, the whole when implemented forming a screw; and that
- the helical element is a filled solid helically shaped comprising at least one core (31) and a thread (32);
- said rod (2) comprises on its outer surface, as many guide elements in the form of an helical groove (4) as helical elements (3), said guide elements in the form of helical groove (4) each having an opening over all or part of their length in the direction of the bone element, and
- each of said at least one helical element (3) comprises a core (31) of shape and dimensions complementary to the shape and dimensions of the at least one guide element with an helical groove shape (4) in order to be able to be inserted and slide in said groove (4) and an anchoring element present over all or part of the length of the core (31) and protruding through the opening in the direction of the bone element.

2. Surgical implant (1) according to claim 1 **characterized in that** at least one of the anchoring elements is a thread (32) .

3. Surgical implant (1) according to any one of claims 1 or 2 **characterized in that** the depth of the at least one helical groove (4) is not constant all along its length.

4. Surgical implant (1) according to any one of claims 1 to 3, **characterized in that** the rod (2) is made of one or more biocompatible materials that are flexible.

5. Surgical implant (1) according to any one of claims 1 to 4, **characterized in that** only some of the portions of the rod (2) are flexible.

6. Surgical implant (1) according to any one of claims 1 to 5, **characterized in that** it has a separate blocking device (5) of the at least on helical element (3) in a desired position on the rod (2).

7. Surgical implant (1) according to any one of claims 1 to 6, **characterized in that** it presents at least one hooking device (6) allowing the insertion and removal of the rod (2) and of the at least one helical element (3) by the surgeon using suitable ancillary instruments.

8. Surgical implant (1) according to any one of the preceding claims, **characterized in that** its developed length is between 35 mm and 140 mm and is inscribed in an arc of a circle between 15 degrees and 90 degrees.

9. Surgical implant (10) for fixing at least one two adjacent vertebrae of a patient, **characterized in that** it is composed of
- a curved rod (11) that has at least one helical groove (13), said curved rod (11) being intended to be inserted first into a borehole made in the at least two vertebrae, and
- at least one separate helical element (12) intended to be inserted secondly on said rod within the at least one helical groove (13), and the core (17) of which has a shape complementary to the shape of the helical groove (13),
the whole when implemented forming a screw.

10. Surgical implant (10) according to claim 9, **characterized in that** the curved rod (11) further presents a distal end that carries a head (16) and a proximal end equipped with a hooking device (14); and that the at least one helical element (12) further presents a thread (18) and its proximal end is equipped with a hooking device (19); the at least one helical element (12) being intended to be inserted in rotation on the rod once put in place.

11. The surgical implant (10) for fixing at least two adjacent vertebrae of a patient according to any one of claims 9 or 10, **characterized in that** it is composed of
- a curved rod (11) that presents two helical grooves (13), said curved rod (11) being intended to be inserted first in a borehole made in the at least two vertebrae, and
- two separate helical elements (12) intended to be inserted secondly on said rod within the two helical grooves (13), and the cores (17) of which present a shape that is complementary to the shape of the helical grooves (13),
the whole when implemented forming a screw.

12. Surgical implant (10) according to any one of claims 9 to 11, **characterized in that** its developed length is between 35 mm and 140 mm and is inscribed in an arc of a circle between 15 degrees and 90 degrees.
